# EUROPEAN PATENT APPLICATION

(11) **EP 0 702 960 A1**
(43) Date of publication of application: **27.03.1996**
(21) Application number: 95910003.3
(22) Date of filing: 28.02.1995
(51) Int. Cl.: A61K 39/395

(54) **LIQUID GLOBULIN COMPOSITION FOR INTRAVENOUS INJECTION, PROCESS FOR PRODUCING THE SAME, AND METHOD OF INHIBITING GLOBULIN DIMER INCREASE IN SAID COMPOSITION**

(30) Priority: 28.02.1994 JP 29266/94
(71) Applicant: THE GREEN CROSS CORPORATION, Osaka-shi Osaka 541 (JP)
(72) Inventor: HIRAO, Yutaka, The Green Cross Corporation, 2-chome Hirakata-shi, Osaka 573 (JP); NAKAJIMA, Tsunetaka, The Green Cross Corporation, 2-chome, Hirakata-shi, Osaka 573 (JP); TAKECHI, Kazuo, The Green Cross Corporation, 2-chome, Hirakata-shi, Osaka 573 (JP)
(74) Representative: VOSSIUS & PARTNER
(86) International application number: JP9500315
(87) International publication number: WO9522990

(57) **Abstract**

A liquid globulin composition for intravenous injection comprising a chemically unmodified complete-molecule γ-globulin of which the globulin dimer content is at most 7 w/w % of the total content of the γ-globulin even after being left to stand at room temperature for at least seven months, the globulin oligomer content is preferably at most 1 w/w %, and either the pH is 5.5±0.2 or the conductance is at most 1mmho (at 8°C). The method of inhibiting globulin dimer increase in this composition comprises leaving the composition to stand either at 15-30 °C for one to ten days or at 37-56 °C for one to two hours.

## Description

### Technical Field

The present invention relates to a liquid globulin composition for intravenous injection having a reduced globulin dimer content, a process for producing said composition, and a method of inhibiting increase of globulin dimer in a liquid globulin composition for intravenous injection.

### Background Art

γ-Globulin preparations comprising, as a main component, particularly IgG from γ-globulins, which are the components of plasma proteins, have been widely used for preventing and treating various infectious diseases.

γ-Globulin is unstable in a solution state and conventionally formulated into lyophilized preparations. Liquid preparations are advantageous as compared to dry preparations in that they need not be dissolved in distilled water for injection and the like and can be administered easily. On the other hand, they have inferior stability, so that the development of a liquid globulin composition for intravenous injection, which is stable in a solution state, has been heretofore desired.

For example, Japanese Patent Unexamined Publication No. 192724/1988 (USP 4876088, EP 278422) proposes a liquid globulin composition for intravenous injection stable in a solution state, which is obtained by combining a low conductivity, a pH of 5.5±0.2 and the use of sorbitol as a stabilizer.

It has also been known that the agglutination of γ-globulin, in particular, denaturing of γ-globulin during fractionation enhances complement fixation of γ-globulin which is called anticomplementary activity, and administration thereof to human beings induces a lowered level of serum complement.

Japanese Patent Unexamined Publication No. 43914/1983 (USP 4396608, USP 4499073, EP 73371) discloses an immune serum globulin solution having an ionic strength of less than about 0.001 and a pH of 3.5-5.0 to provide an immunoglobulin composition substantially free of immunoglobulin agglutinate and having an immunoglobulin monomer content greater than about 90%.

Japanese Patent Unexamined Publication No. 8340/1988 (USP 4762714, USP 4948877, EP 240856) discloses a method of preparing immune serum globulin substantially free of acquired viruses, comprising preparing an immune serum globulin from human plasma as a starting material by cold ethanol fractionation at a pH of about 5.4 or below and preserving the obtained immune serum globulin at a pH of about 4.25 or below for at least about 3 days or at a pH of about 6.8 or below at at least 45°C, so that it will not contain infectious retroviruses. This invention aims at inactivating retroviruses and no report has been given that the inhibition of immunoglobulin agglutinate was found in the formulated immune serum globulin preparation.

As described supra, globulin is inherently an unstable protein and one of the difficulties associated with globulin is its poor stability upon being prepared into a liquid composition. For an improved stability thereof, a safe and effective method for inhibiting agglutination of globulin, namely, inhibiting increase of globulin dimer is desired. In addition, a globulin composition for intravenous injection which is free from increase in globulin dimer content and stable during a long-term storage, namely, that having a reduced content of globulin dimer is demanded.

### Disclosure of the Invention

It is an object of the present invention to provide a liquid globulin composition for intravenous injection having an extremely small globulin dimer content, and a process for producing said composition. The present invention further aims at providing a method of inhibiting increase of globulin dimer in a liquid globulin composition for intravenous injection.

The present inventors have conducted intensive studies from the above-mentioned aspects and found a composition and a method capable of achieving the above objects safely and effectively, that is, without exerting adverse influences on other properties of globulin.

The present invention provides the following.
(1) A liquid globulin composition for intravenous injection, comprising chemically unmodified complete molecule type γ-globulin, which has a globulin dimer content to the total amount of chemically unmodified complete molecule type γ-globulin, of not more than 7 w/w%, even after standing the composition at room temperature for at least 7 months.
   This composition includes those containing a low molecular weight compound of globulin monomer in a proportion of not more than 1 w/w% relative to the total amount of the chemically unmodified complete molecule type γ-globulin, those having a pH of 5.5±0.2 and those having a conductivity of not more than 1 mmho (converted to 8°C).
(2) A process for producing a liquid globulin composition for intravenous injection, having a reduced globulin dimer content, which comprises standing a liquid globulin composition for intravenous injection comprising chemically unmodified complete molecule type γ-globulin, at 15-30°C for 1 to 10 days or at 37-56°C for 1-2 hours.
(3) A method of inhibiting increase of globulin dimer in a liquid globulin composition for intravenous injection comprising chemically unmodified complete molecule type γ-globulin, comprising adjusting the pH thereof to 3.4-5.0 and standing same.
(4) A method of inhibiting increase of globulin dimer in a liquid globulin composition for intravenous injection comprising chemically unmodified complete molecule type γ-globulin, comprising standing the composition at 15-30°C for 1 to 10 days or at 37-56°C for 1-2 hours.

### Detailed Description of the Invention

The γ-globulin contained in the liquid globulin composition for intravenous injection of the present invention is of chemically unmodified complete molecule type. By chemically unmodified complete molecule type is meant a γ-globulin having the properties of;
① being intact and free from any modification or changes, and therefore, without γ-globulin fragments Fab, F(ab')₂, Fc and the like,
② being free from lowering of antibody titer and lowering of antibody spectrum, and
③ having an anti-complementary activity (complement fixation) sufficiently lower than 20 units (CH₅₀) which is regarded to be safe by the Japanese biological products standard.

(1) Liquid globulin composition for intravenous injection
   The liquid globulin composition for intravenous injection of the present invention comprises globulin dimer in the chemically unmodified complete molecule type γ-globulin in a proportion of not more than 7 w/w%, preferably not more than 6 w/w%, and more preferably not more than 4 w/w%, and maintains such level or a lower level even after standing at room temperature for at least 7 months.
   While there is no particular limitation imposed on the pH of said liquid globulin composition for intravenous injection, it is preferably in the range of from 3.4 to 8 in view of the safety region for intravenous injection, and from 5 to 6, particularly 5.5±0.2 for inhibiting the globulin monomer from becoming a low molecular weight compound.
   While the conductivity of this composition is not particularly limited, it is preferably low and more preferably not more than 1 mmho (converted to 8°C).
   Moreover, the γ-globulin contained in the liquid globulin composition for intravenous injection of the present invention preferably has a reduced content of a low molecular weight compound of globulin monomer. More preferably, the liquid globulin composition for intravenous injection comprises a low molecular weight compound of globulin monomer (hereinafter referred to as low molecular weight globulin) in a proportion of not more than about 1 w/w% relative to the total amount of the chemically unmodified complete molecule type γ-globulin contained in the liquid globulin composition for intravenous injection.
   The process for producing said liquid globulin composition for intravenous injection is not particularly limited, and can be, for example, ① a method comprising standing a liquid globulin composition for intravenous injection comprising chemically unmodified complete molecule type γ-globulin prepared according to a conventional method or the method disclosed in Japanese Patent Unexamined Publication No. 20415/1978, 43194/1983, 42336/1985 or 192724/1988, or a commercially available chemically unmodified complete molecule type γ-globulin preparation for intravenous injection (hereinafter these are sometimes also referred to as starting composition), at 15-30°C for 1 to 10 days or at 37-56°C for 1-2 hours, or ② a method comprising adjusting the pH of a liquid composition of chemically unmodified complete molecule type γ-globulin obtained by the above-mentioned method or a commercially available chemically unmodified complete molecule type γ-globulin preparation for intravenous injection, to 3.4-5, or ③ a method comprising preserving the composition at room temperature.
   When the starting composition to be used contains the globulin dimer in a proportion of more than 7 w/w%, the liquid composition of the present invention can be obtained by the aforementioned method ① or ② , that is, by adjusting its pH to 3.4-5, followed by standing the composition for at least one day, preferably 3 days or more.
   When a starting composition to be used contains the globulin dimer in a proportion of not more than 7 w/w%, the liquid composition of the present invention having a further reduced globulin dimer content can be obtained by the method ① . It is also possible to prepare a stable liquid composition of the present invention by the method ② or ③ .
   When the method ① is used, the composition is preferably left standing at room temperature or heat-treated at pH 5-6, particularly at pH 5.5±0.2. It is generally preferable to preserve the composition at low temperature for a long time and subject same to the method ① immediately before clinical use.
   When the method ② is used, a low temperature (0-10°C, preferably not more than 5°C) is preferably employed. According to the method ② , a liquid composition of chemically unmodified complete molecule type γ-globulin can be obtained while maintaining a reduced globulin dimer content during a long-term preservation [6-36 months (preferably 7 months or more) at low temperature] and simultaneously inhibiting the globulin monomer from becoming a low molecular weight compound.
   When the method ③ is used, a pH of 5-6, preferably 5.5±0.2 is used. According to the method ③ , a liquid composition of chemically unmodified complete molecule type γ-globulin can be obtained while maintaining a reduced globulin dimer content during a long-term preservation [6-36 months (preferably 7 months or more)] and simultaneously inhibiting the globulin monomer from becoming a low molecular compound.
   The liquid γ-globulin composition of the present invention may comprise, besides the chemically unmodified complete molecule type γ-globulin, a pharmaceutically acceptable carrier or additive. Examples thereof include polyethylene glycol having a molecular weight of 1,000 - 10,000 at 0.1-1 w/v%, human serum albumin at 0.5-5 w/v%, mannitol at 1-10 w/v%, combinations thereof, sorbitol at 1-20 w/v% and sucrose at 1-20 w/v%.
(2) Process for producing liquid globulin composition for intravenous injection
   The process for producing the liquid globulin composition for intravenous injection of the present invention comprises standing the liquid globulin composition for intravenous injection comprising chemically unmodified complete molecule type γ-globulin, at 15-30°C for 1-10 days, preferably at 25°C for 1-10 days, or at 37-56°C for 1-2 hours, preferably 37°C for 2 hours or 56°C for one hour.
   The "liquid globulin composition for intravenous injection comprising chemically unmodified complete molecule type γ-globulin" before standing at 15-30°C or 37-56°C can be prepared by the method disclosed in, for example, Japanese Patent Unexamined Publication No. 20415/1978, 43194/1983, 42336/1985 or 192724/1988, or using a commercially available chemically unmodified complete molecule type γ-globulin preparation for intravenous injection. The liquid globulin composition before allowing to stand may comprise globulin dimer in a proportion of not less than 7 w/w%, preferably from 10 to 15 w/w% relative to the total amount of γ-globulin. While the pH of said liquid composition is not limited, it is within the range of from 3.4 to 8 in view of safety region for intravenous injection, and from 5 to 6, particularly 5.5±0.2 for inhibiting globulin monomer from becoming a low molecular weight compound.
   The liquid globulin composition before allowing to stand may comprise, besides the chemically unmodified complete molecule type γ-globulin, a pharmaceutically acceptable carrier or additive. Examples of the additive include polyethylene glycol having a molecular weight of 1,000 - 10,000 at 0.1-1 w/v%, human serum albumin at 0.5-5 w/v%, mannitol at 1-10 w/v%, combinations thereof, sorbitol at 1-20 w/v% and sucrose at 1-20 w/v%. While the conductivity of the liquid globulin composition is not particularly limited, it is preferably low and more preferably not more than 1 mmho (converted to 8°C).
   When to stand the composition at 15-30°C or 37-56°C is not particularly limited. It may be done consecutively after preparation of the liquid globulin composition for intravenous injection according to the above-mentioned methods, or immediately before clinical use thereof. It is preferably done immediately before clinical use thereof.
   According to the process of the present invention, a liquid globulin composition comprising chemically unmodified complete molecule type γ-globulin, which has a reduced globulin dimer content, can be prepared. More specifically, a liquid γ-globulin composition can be prepared, wherein globulin dimer is contained in a proportion of not more than 7 w/w%, preferably not more than 6 w/w%, and more preferably not more than 4 w/w% relative to the total amount of the chemically unmodified complete molecule type γ-globulin. By allowing the composition to stand at pH of 5-6, a liquid γ-globulin composition inhibiting globulin monomer from becoming a low molecular weight compound (preferably a composition comprising a low molecular weight globulin in a proportion of not more than 1 w/w% relative to the total amount of the chemically unmodified complete molecule type γ-globulin) can be prepared. The liquid γ-globulin composition obtained by the method of the present invention can maintain the globulin dimer content of at least not more than 7 w/w% during a long-term preservation at room temperature for 6-36 months, preferably 7 months or more.
(3) Method for inhibiting increase of globulin dimer in liquid globulin composition for intravenous injection
   The method for inhibiting increase of globulin dimer of the present invention comprises ① standing a liquid globulin composition for intravenous injection comprising chemically unmodified complete molecule type γ-globulin, at a pH of 3.4-5 or ② standing a liquid globulin composition for intravenous injection comprising chemically unmodified complete molecule type γ-globulin, at 15-30°C for 1 to 10 days, preferably at 25°C for 1 to 10 days, or at 37-56°C for 1-2 hours, preferably at 37°C for 2 hours or 56°C for one hour.
   The subject "liquid globulin composition for intravenous injection comprising chemically unmodified complete molecule type γ-globulin" is not particularly limited, and is exemplified by liquid globulin composition prepared by the method described in the aforementioned publications and globulin preparations for intravenous injection. Again, the composition may comprise, besides chemically unmodified complete molecule type γ-globulin, a pharmaceutically acceptable carrier or additive.

### ① Method for allowing to stand at pH 3.4-5

The globulin dimer content in the subject "liquid globulin composition for intravenous injection comprising chemically unmodified complete molecule type γ-globulin" is not subject to particular limitation. When the liquid globulin composition for intravenous injection comprises the globulin dimer in a proportion of more than 7 w/w% relative to the total amount of γ-globulin, the composition is stood at pH 3.4-5, preferably 3.4-4.6 for at least one day, preferably at least three days to reduce the globulin dimer content to not more than 7 w/w%, preferably not more than 6 w/w%, and more preferably not more than 4 w/w%, which ranges being the pharmaceutically safe range. When the liquid globulin composition for intravenous injection comprises the globulin dimer in a proportion of not more than 7 w/w% relative to the total amount of γ-globulin, the composition is stood at pH 3.4-5, preferably 3.4-4.6 to inhibit increase of the globulin dimer.

The pH of the liquid globulin composition for intravenous injection can be adjusted by the use of a pharmaceutically acceptable acid or base conventionally used for adjusting pH, such as hydrochloric acid, acetic acid and citric acid.

While the temperature at which the composition is stood is not particularly limited, it is preferably from 0°C to 10°C. At this temperature range, it is possible to inhibit globulin monomer from becoming a low molecular weight compound, thus enabling stable preservation of γ-globulin at low temperatures for 6-36 months, preferably 7 months or longer.

### ② Method for standing at 15-30°C for 1 to 10 days or at 37-56°C for 1-2 hours

The content of globulin dimer in the "liquid globulin composition for intravenous injection comprising chemically unmodified complete molecule type γ-globulin" which is the subject of this method is not particularly limited, and the globulin dimer may be contained in a proportion of not less than 7 w/w%, preferably 10-15 w/w% relative to the total amount of γ-globulin.

The pH of the liquid globulin composition to be stood is not particularly limited and can be within a pH range commonly used for intravenous injections. Such liquid globulin composition for intravenous injection is stood at 15-30°C for 1 to 10 days, preferably at 25°C for 1 to 10 days, or at 37-56°C for 1-2 hours, preferably at 37°C for 2 hours or 56°C for one hour, whereby the content of the globulin dimer in the liquid globulin composition for intravenous injection can be reduced and increase thereof can be inhibited.

The present method is applied to a liquid globulin composition for intravenous injection preferably adjusted to have a pH of 5-6, more preferably 5.5±0.2. Under these pH conditions, reduction of the globulin dimer, inhibition of increase of the globulin dimer and inhibition of globulin monomer from becoming a low molecular weight compound can be simultaneously achieved, whereby γ-globulin can be preserved at room temperature in a stable condition for 6-36 months, preferably 7 months or longer.

The present invention is described in more detail in the following by illustrative Examples, Experimental Examples and Reference Examples. It should be understood that the present invention is not limited to these Examples and Experimental Examples.

### Reference Example 1

Chemically unmodified complete molecule type γ-globulin for intravenous injection, obtained from Cohn's fractions II + III was prepared into a 5% solution using distilled water. The content of globulin dimer in this solution was 10-14%.

### Reference Example 2

A paste (1 kg) of Cohn's fractions II + III was suspended in 10 L of distilled water and adjusted to pH 5.5. The suspension was centrifuged and supernatant was recovered. Sorbitol (50 g) was added per 100 ml of the supernatant to the final concentration of 33 w/v% and the mixture was heat-treated at 60°C for 10 hours.

After the heat treatment, pH was adjusted to 5.5 and PEG #4000 was added to the final concentration of 6%, followed by cetrifugation at 2°C. The supernatant obtained was adjusted to pH 8.0 with 1N sodium hydroxide. PEG #4000 was added to the final concentration of 12%, and the mixture was centrifuged at 2°C to give chemically unmodified complete molecule type γ-globulin fractions in the precipitated fractions.

The obtained fractions were dissolved in distilled water and DEAE-Sephadex was added to 100 ml of the γ-globulin solution at a ratio of 1 ml per 50 ml of the solution. The mixture was subjected to contact treatment for about one hour at 0-4°C, ultracentrifuged at 7,000 rpm for about 20 minutes to recover supernatant (γ-globulin solution).

This chemically unmodified complete molecule type γ-globulin solution was adjusted into a 5 w/v% solution using distilled water and sterilized by filtration to give a liquid γ-globulin composition for intravenous injection.

### Example 1

The liquid composition obtained in Reference Example 1 was adjusted to pH 5.5 with acetic acid, heated at 37°C for 2 hours, and changes in the content of globulin dimer relative to the total amount of γ-globulin were examined. As a result, the content of the globulin dimer which was 12.6 w/w% before heating decreased to 6.8 w/w% after heating.

### Example 2

The liquid composition obtained in Reference Example 1 was adjusted to pH 5.5 with acetic acid, left standing for 24 hours at 25°C, and changes in the content of globulin dimer were examined. As a result, the content of the globulin dimer which was 11.2 w/w% before standing decreased to 7 w/w% after standing.

### Example 3

The liquid composition obtained in Reference Example 1 was adjusted to pH 5.0 with acetic acid, left standing for 24 hours at 25°C, and changes in the content of globulin dimer were examined. As a result, the content of the globulin dimer which was 10.4 w/w% before standing decreased to 5 w/w% after standing.

### Example 4

The liquid composition obtained in Reference Example 1 was adjusted to pH 5.5 with acetic acid, heated at 56°C for one hour, and changes in the content of globulin dimer were examined. As a result, the content of the globulin dimer which was 14.6 w/w% before standing decreased to 1.7 w/w% after standing.

### Example 5 : Long-term preservation at acidic pH

The liquid composition prepared in Reference Example 2 was adjusted to pH 4 with acetic acid, and the mixture was stored for one year at a low temperature of 5°C. The content of the globulin dimer was not more than 7 w/w% both immediately after adjusting to pH 4 and after the long-term preservation.

### Example 6 : Long-term preservation at room temperature

The liquid composition prepared in Reference Example 2 was adjusted to pH 5.5 with acetic acid, and the mixture was stored for one year at room temperature. The content of the globulin dimer was not more than 7 w/w% both immediately after adjusting to pH 5.5 and after the long-term preservation.

### Example 7 : With heat treatment after long-term preservation at low temperature

A polyethylene glycol-treated immunoglobulin preparation for intravenous injection [lyophilized powder preparation containing immunoglobulin 500 mg, polyethylene glycol (#4000) 50 mg, human serum albumin 100 mg and mannitol 200 mg] was dissolved in 10 ml of water for injection and used as a liquid globulin composition. The composition was adjusted to pH 5.5 with acetic acid, and stored for one year at a low temperature of 5°C. Then, the composition was heated at 37°C for 2 hours and put to clinical use. The content of the globulin dimer was not more than 7 w/w% both immediately after adjusting to pH 5.5 and after the heat treatment.

### Examples 8-12

The liquid composition (globulin dimer content 11 w/w%) obtained in Reference Example 1 was adjusted to pH 3.4-5 with acetic acid, and left standing for 30 days at 5°C. The composition after standing was examined for the globulin dimer content relative to the total amount of γ-globulin. The results are shown in Table 1.

**Table 1**

| Example | pH | Globulin dimer content (w/w%) |
|---|---|---|
| 8 | 3.4 | 1 |
| 9 | 3.8 | 2 |
| 10 | 4.2 | 4 |
| 11 | 4.6 | 6 |
| 12 | 5.0 | 9 |

### Experimental Example 1 : Method for decreasing, and inhibiting increase of globulin dimer - 1

The liquid composition obtained in Reference Example 1 (globulin dimer content 11 w/w%) was adjusted to pH 3.0-70 with acetic acid and left standing for 30 days at 5°C. Changes in globulin dimer content was examined with the lapse of time. The globulin dimer content after standing for 30 days are shown in Table 2.

**Table 2**

| pH | Globulin dimer content (%) |
|---|---|
| 3.0 | 10 |
| 3.4 | 1 |
| 3.8 | 2 |
| 4.2 | 4 |
| 4.6 | 6 |
| 5.0 | 9 |
| 5.2 | 10 |
| 6.0 | 13 |
| 7.0 | 15 |

The content of the globulin dimer in the liquid composition decreased with the lapse of time in the pH range of from 3.4 to 5.0. On the other hand, the content of the globulin dimer did not decrease in the pH range of 3.0 and 5.2-7.0. In particular, the content of the globulin dimer increased with time at pH 7.0.

### Experimental Example 2 : Method for decreasing, and inhibiting increase of globulin dimer - 2

The globulin dimer content in Sample Nos. 1-4 obtained by treating the liquid composition obtained in Reference Example 1 (globulin dimer content 11 w/w%), under the conditions shown in Table 3 below was examined. The results are shown in Table 3.

**Table 3**

| Sample No. | Treatment conditions | | | Globulin dimer content (w/w%) |
|---|---|---|---|---|
| | pH | Temperature | Treated period | |
| 1 | 5.5 | 15°C | 10 days | not more than 7 |
| 2 | 5.5 | 30°C | 24 hours | not more than 7 |
| 3 | 6.0 | 25°C | 24 hours | not more than 7 |
| 4 | 5.5 | 45°C | 1 hour | not more than 7 |

### Experimental Example 3

The composition of Sample Nos. 1-4 obtained by preserving the preparation obtained in Reference Example 1, under the conditions shown in Table 4 below was determined by HPLC and the pattern thereof was analyzed.

**Table 4**

| Sample No. | 1 | 2 | 3 | 4 |
|---|---|---|---|---|
| pH | 5.0 | 5.0 | 5.5 | 5.5 |
| temperature | 25°C | 25°C | 25°C | 5°C |
| preservation period | 10 days | 50 days | 10 days | 6 months |
| globulin dimer (w/w%) | 4.1 | 4.6 | 6.9 | 10.2 |
| globulin monomer (w/w%) | 94.3 | 90.0 | 93.1 | 89.8 |
| low molecular weight compound (w/w%) | 1.6 | 5.4 | - | - |

Sample Nos. 1 and 2 which had been adjusted to pH 5.0 and preserved at 25°C showed a decrease in globulin dimer content to not more than 7 w/w%, whereas low molecular weight globulin appeared. Sample No. 3 which had been preserved at 25°C for 10 days had a globulin dimer content of 6.9 w/w% and was accompanied by no appearance of low molecular weight globulin. Sample No. 4 which had been stored at 5°C for 6 months did not show appearance of low molecular weight globulin, whereas it contained globulin dimer at a proportion of 10.2 w/w%.

## Claims

1. A liquid globulin composition for intravenous injection comprising chemically unmodified complete molecule type γ-globulin, having a globulin dimer content of not more than 7 w/w% relative to the chemically unmodified complete molecule type γ-globulin, even after standing at room temperature for at least 7 months.

2. The liquid globulin composition for intravenous injection of Claim 1, wherein a low molecular weight globulin content relative to the chemically unmodified complete molecule type γ-globulin is not more than 1 w/w%.

3. The liquid globulin composition for intravenous injection of Claim 1 or Claim 2, having a pH of 5.5±0.2.

4. The liquid globulin composition for intravenous injection of any one of Claims 1 to 3, having a conductivity of not more than 1 mmho (converted to 8°C).

5. A process for producing a liquid globulin composition for intravenous injection having a reduced globulin dimer content, comprising standing a liquid globulin composition for intravenous injection comprising chemically unmodified complete molecule type γ-globulin, at 15-30°C for 1 to 10 days or at 37-56°C for 1-2 hours.

6. A method for inhibiting increase of globulin dimer in a liquid globulin composition for intravenous injection comprising chemically unmodified complete molecule type γ-globulin, which comprises standing the composition at pH 3.4-5.0.

7. A method for inhibiting increase of globulin dimer in a liquid globulin composition for intravenous injection comprising chemically unmodified complete molecule type γ-globulin, comprising standing the composition at 15-30°C for 1 to 10 days or at 37-56°C for 1-2 hours.
